(19) Europäisches Patentamt
European Patent Office
Office européen des brevets

(11) Veröffentlichungsnummer: **0 049 852**
**B1**

(12) # EUROPÄISCHE PATENTSCHRIFT

(45) Veröffentlichungstag der Patentschrift: **21.08.85**

(51) Int. Cl.[4]: **A 61 K 9/48**, A 61 K 31/557

(21) Anmeldenummer: **81107988.8**

(22) Anmeldetag: **06.10.81**

(54) **Pharmazeutisches Präparat und seine Herstellung.**

(30) Priorität: **09.10.80 US 195575**

(43) Veröffentlichungstag der Anmeldung: **21.04.82 Patentblatt 82/16**

(45) Bekanntmachung des Hinweises auf die Patenterteilung: **21.08.85 Patentblatt 85/34**

(84) Benannte Vertragsstaaten: **AT BE CH DE FR GB IT LI LU NL SE**

(56) Entgegenhaltungen:
**DE - A - 2 216 717**
**FR - A - 2 209 544**
**GB - A - 2 012 168**
**US - A - 2 889 252**
**US - A - 3 932 634**
**US - A - 4 052 446**

**Dr. H.P. FIEDLER: "Lexikon der Hilfsstoffe für Pharmazie, Kosmetik und angrenzende Gebiete", Band 9, 1971, Seite 68, Der pharmazeutische Betrieb, Ausg. Cantor, Aulendorf, DE.**

(73) Patentinhaber: **F. HOFFMANN-LA ROCHE & CO. Aktiengesellschaft, CH-4002 Basel (CH)**

(72) Erfinder: **Gallo-Torres, Hugo Enrique, 215 East Mount Pleasant Avenue, Livingston New Jersey 07039 (US)**
Erfinder: **Shah, Navnit H., 203 Beverly Hill Road, Clifton New Jersey 07012 (US)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

**EP 0 049 852 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Natürliche Prostaglandine sind wirksame Hemmer der basalen und der stimulierten Magensäureproduktion. An der Pathogenese des peptischen Ulcers ist ein Mangel an Prostaglandinen des E-Typs beteiligt. Prostaglandine sind allgemein therapeutisch nützlich, nicht nur wegen ihrer antisekretorischen Aktivität, sondern auch wegen ihrer antihypertensiven Aktivität; und sie sind als cardiovaskuläre Mittel, als Mittel zur Einleitung von Wehen und zum Schwangerschaftsabbruch verwendet worden.

Eine Schwierigkeit, Prostaglandine therapeutisch wirksam anzuwenden, ist in ihrer schlechten Bioverfügbarkeit bezüglich ihrer antisekretorischen Aktivität und ihrer Instabilität in pharmazeutischen Formulierungen begründet.

Die vorliegende Erfindung betrifft ein oral verabreichbares pharmazeutisches Präparat in Form einer Weichgelatinekapsel. Die Kapsel enthält ein organisches Lösungsmittel, die ein darin enthaltenes Prostaglandin bei der oralen Verabreichung bioverfügbar macht. Die Kapsel enthält weiterhin als Wirkstoff eine therapeutisch wirksame Menge eines Prostaglandins sowie eine wirksame Menge Ascorbylpalmitat, das das Prostaglandin stabilisiert.

Der Wirkstoff ist ein Prostaglandin der allgemeinen Formel

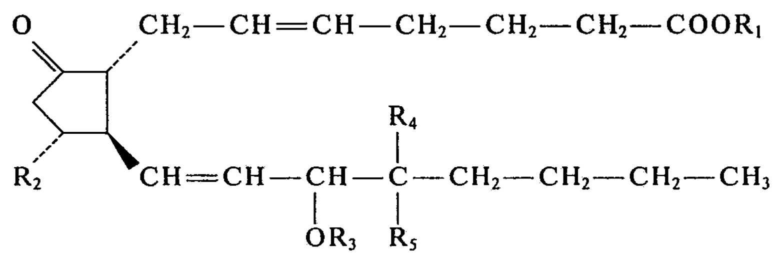

(I)

worin

$R_1$ Wasserstoff oder $C_{1-7}$-Alkyl
$R_2$ Wasserstoff oder $C_{1-7}$-Alkyl
$R_3$ Wasserstoff oder Acetyl;
$R_4$ Wasserstoff oder $C_{1-7}$-Alkyl und
$R_5$ Wasserstoff, $C_{1-7}$-Alkyl oder Fluor darstellt.

Prostaglandine der obigen allgemeinen Formel, die für die erfindungsgemäßen Kompositionen besonders geeignet sind, sind Nat-11R-methyl-16R-fluor-15R-hydroxy-9-oxoprosta-cis-5-trans-13-dien-1-säure, Nat-11-R,16,16-trimethyl-15R-hydroxy-9-oxoprosta-cis-5-trans-13-dien-1-säure und dessen Methylester und (8R, 11R, 12S, 15R, 5Z, 13E)-15-(Acetoxy)-11,16,16-trimethyl-9-oxoprosta-cis-5-trans-dien-1-säure. Die Prostaglandine der allgemeinen Formel I in denen $R_1$ Wasserstoff ist, sind bekannt. Verbindungen der Formel I, in denen $R_3$ Acetyl ist können aus den entsprechenden Hydroxyverbindungen ($R^3=H$) durch Behandlung mit einem Acetylierungsmittel, wie dies eingehender in der EP-A1-0 049 853 beschrieben ist, hergestellt werden.

Der bevorzugte Konzentrationsbereich für das Prostaglandin beträgt 0,025 bis 2 Gewichtsprozent im Gemisch (Lösungsmittel, Prostaglandin und Ascorbylpalmitat), das in der erfindungsgemäßen Kapsel enthalten ist, wobei ein Gehalt von 0,25 bis 0,5% besonders bevorzugt ist.

Der Ausdruck $C_{1-7}$-Alkyl betrifft geradkettige und verzweigte Alkylgruppen mit $C_{1-7}$-Atomen wie Methyl, Äthyl und Propyl.

Die das Gemisch von Lösungsmittel, aktivem Inhaltsstoff und Stabilisator enthaltende Weichgelatinekapsel wirkt als pharmazeutischer Träger für den aktiven Inhaltsstoff bei der oralen Verabreichung der Kapsel. Die erfindungsgemäßen Kapseln können mit konventionellen Geräten und Verfahren hergestellt werden. Solche Verfahren sind insbesondere in den US-Patentschriften 2 899 361 und 2 928 128, beschrieben.

Bisher wurde allgemein angenommen, daß die Bioverfügbarkeit von oral verabreichten Prostaglandinen dadurch erreicht werden kann, daß man das Prostaglandin in Lösungsmitteln wie Tris-Äthanol oder Phosphatpuffern löst. Erfindungsgemäß wurde überraschend gefunden, daß Polyoxyäthylensorbitanfettsäureester (PGSE) oder Polyäthylenglykole (PEG) oder Kombinationen davon wirksamere Lösungsmittel als Tris-Äthanol oder Phosphatpuffer darstellen, um das Prostaglandin bioverfügbar zu machen. Die verbesserte Bioverfügbarkeit der Prostaglandine verbessert wiederum ihre pharmakologische Wirksamkeit.

Besonders bevorzugte PGSE sind die, die sich von aliphatischen Verbindungen mit 12—18 C-Atomen ableiten. Die PGSE sind Polyoxyäthylenpolymere mit 18—20 Äthylenoxidgruppen, in denen das Sorbitan mit einer Fettsäure wie Stearinsäure, Ölsäure oder Plamitinsäure verestert ist. Besonders bevorzugte PGSE sind Polysorbat 80 Polyäthylen (20) Sorbitan-monooleat) und Polysorbat 60 (Polyoxyäthylen (29) Sorbitan-monostearat. Bevorzugte Polyäthylenglykole sind die mit einem Molekulargewicht von 200—6000, besonders bevorzugt sind die mit Molekulargewichten von 400 (PEG 400) oder 6000 (PEG 6000). PGSE und PEG haben sich überraschenderweise als besonders geeignet erwiesen,

die Bioverfügbarkeit, die Potenz und die Wirkungsdauer der Prostaglandine bei oraler Verabreichung an Lebewesen (siehe Beispiel 10) zu erhöhen. Der Prozentsatz von PEG und PGSE oder Kombinationen davon in dem in der Kapsel enthaltenen Gemisch kann von 90,0 bis 99,9 Gewichtsprozent variieren, besonders bevorzugt sind 97—99,5 Gewichtsprozent.

Das erfindungsgemäß angewandte Lösungsmittel kann aus Kombinationen von PGSE und PEG in jedem Verhältnis bestehen, bei dem eine Flüssigkeit resultiert oder bei dem das Gemisch durch konventionelles Erwärmen verflüssigt werden kann.

Der Stabilisator für die Prostaglandine in dem erfindungsgemäßen Präparat ist Ascorbylpalmitat. Der bevorzugte Konzentrationsbereich für den Stabilisator beträgt 0,05 bis etwa 5 Gewichtsprozent des gesamten Gemsiches, das in der Kapsel enthalten ist, wobei ein Gehalt von 0,2 bis 2% bevorzugt ist. Dieser Bereich hat sich als wirksame Menge zur chemischen Stabilisierung des aktiven Inhaltsstoffs erwiesen. Der chemische Effekt des Stabilisators bewirkt eine Verlängerung der Stabilität der Prostaglandine von einigen Wochen bis zu mehreren Jahren.

Die erfindunsgemäßen Präparate können neben dem Ascorbat auch andere Stabilisatoren enthalten, beispielsweise Bisulfite, butyliertes Hydroxyanisol (BHA), butyliertes Hydroxytoluol (BHT), butyliertes Hydrochinon, Thiodipropionsäure, Dilaurylthiodipropionat, Ethoxyquin, $\alpha$-Tocopherol, Thioharnstoff, Thioglycerin, Lecithin, Propylgallat, Nordihydroguaijaretsäure, 2-tert-Butyl-hydrochinon und Hydrochinon. Die Menge dieser anderen Stabilisatoren kann bis zu 0,5 Gewichtsprozent des gesamten Kapselinhalts betragen, wobei eine Menge von bis zu 0,2% bevorzugt ist.

Die Menge der in der Gelatinekapsel enthaltenen Prostaglandine kann etwa 0,02 bis 10 mg, vorzugsweise 0,3 bis 2,5 mg betragen.

Eine typische Herstellungsweise für die erfindungsgemäßen Präparate besteht darin, daß man Ascorbylpalmitat und butyliertes Hydroxyanisol flüssigem Polyäthylenglycol bei Raumtemperatur zusetzt. Falls das Polyäthylenglykol bei Raumtemperatur nicht flüssig ist, wird es bis zur Verflüssigung erwärmt. Danach wird das Prostaglandin zugesetzt und gelöst. Das Verfahren wird vorzugsweise unter Stickstoffatmosphäre durchgeführt. Das erhaltene flüssige Gemisch wird dann in Weichgelatinekapseln abgefüllt. Das Abfüllverfahren wie auch das Herstellungsverfahren für die Kapsel kann mit bekannten, konventionellen Methoden vorgenommen werden.

Die nachfolgenden Beispiele erläutern die Erfindung weiter, ohne sie zu beschränken.

## Beispiel 1

0,2 mg BHA und 1,0 mg Ascorbylplamitat werden in 400 mg Polyäthylenglykol-400 bei Raumtemperatur unter Stickstoffatmosphäre gelöst. Die Lösung wird mit 0,25 mg Nat-11R,16,16-Trimethyl-15R-hydroxy-9-oxoprosta-cis-5-trans-13-dien-1-säure (im folgenden auch Trimethyl-$PGE_2$-säure) bei Raumtemperatur unter Stickstoff versetzt. Nachdem alles gelöst ist, wird das erhaltene Gemisch in flüssiger Form in Weichgelatinekapseln abgefüllt.

## Beispiel 2

300 mg PEG 400 und 100 mg PEG 4000 werden unter Stickstoff erwärmt bis sich das Gemisch verflüssigt hat. Danach werden unter Stickstoff 0,1 mg BHA, 0,1 mg BHT und 1,0 mg Ascorbylpalmitat zugesetzt. Nachdem sich alles gelöst hat, werden 0,5 mg Trimethyl-$PGE_2$-säure unter Stickstoff zugefügt und unter Durchmischung gelöst. Die Flüssigkeit wird dann in Weichgelatinekapseln abgefüllt.

## Beispel 3

0,2 mg BHA, 0,2 mg BHT und 1,0 mg Ascorbylpalmitat werden in 400 mg Polysorbat-80 bei Raumtemperatur unter Stickstoff gelöst. Das Gemisch wird unter Stickstoff mit 0,5 mg Trimethyl-$PGE_2$-säure versetzt. Nachdem sich alles gelöst hat, wird die Flüssigkeit in Weichgelatinekapseln abgefüllt.

## Beispiel 4

Ein Gemisch von je 200 mg Polysorbat-60 und Polysorbat-80 wird erwärmt. Das erhaltene flüssige Gemisch wird unter Stickstoff mit 0,2 mg BHA, 1,0 mg $\alpha$-Tocopherol und 2,0 mg Ascorbylpalmitat versetzt. Nachdem alles gelöst ist, werden 0,25 mg Trimethyl-$PGE_2$-säure unter Stickstoff zugesetzt. Nach Durchmischung bis zur vollständigen Lösung wird das erhaltene Gemisch in Weichgelatinekapseln abgefüllt.

## Beispiel 5

| | | | | |
|---|---|---|---|---|
| (8R, 11R, 12S, 15R, 5Z, 13E)-15-(Acetoxy)-11,16,16-trimethyl-9-oxoprosta-5,13-dien-1-säure, mg/Kapsel | 0,25 | 0,1 | 0,50 | 1,0 |
| Polyäthylenglycol 400, mg/Kapsel | 400,0 | 400,0 | 400,0 | 400,0 |
| butyliertes Hydroxyanisol (BHA), mg/Kapsel | 0,2 | 0,2 | 0,2 | 0,2 |
| Ascorbylpalmitat, mg/Kapsel | 1,0 | 1,0 | 1,0 | 1,0 |

Die obigen Formulierungen werden nach dem folgenden Verfahren hergestellt:

Lösen von BHA und Ascorbylplamitat in PEG 400. Zusetzen des aktiven Inhaltsstoffes unter Stickstoffatmosphäre. Abfüllen des erhaltenen Gemisches in flüssiger Form in Weichgelatinekapseln:

## Beispiel 6

| | | | | |
|---|---|---|---|---|
| (8R, 11R, 12S, 15R, 5Z, 13E)-15-(Acetoxy)-11,16,16-trimethyl-9-oxoprosta-5,13-dien-1-säure, mg/Kapsel | 0,025 | 1,0 | 0,50 | 1,0 |
| Polyäthylenglycol 400, mg/Kapsel | 200,0 | 200,0 | 200,0 | 200,0 |
| Polysorbat-80, mg/Kapsel | 200 | 200,0 | 200,0 | 200,0 |
| butyliertes Hydroxyanisol, mg/Kapsel | 0,2 | 0,2 | 0,2 | 0,2 |
| Ascorbylpalmitat, mg/Kapsel | 1,0 | 1,0 | 1,0 | 1,0 |

Die obigen Formulierungen werden nach dem folgenden Verfahren hergestellt:

Lösen von BHA und Ascorbylpalmitat in einem Gemisch von PEG-400 und Polysorbat-80. Zusatz des aktiven Inhaltsstoffes und Lösen unter Stickstoffatmosphäre. Abfüllen des Gemisches in flüssiger Form in Weichgelatinekapseln.

## Beispiel 7

In Stellung 11 tritiierte 11R, 16, 16-Trimethyl-15R-hydroxy-9-oxoprosta-cis-5-trans-13-dien-1-säure wurde in zwei unterschiedlichen Formulierungen (I und II gemäß Tabelle 1) an Ratten verabreicht. Die Ratten hatten einen Katheter in der Pfortader und eine Magenkanüle. Die Konzentration des tritiierten Prostaglandins war so eingestellt, daß 10 µg/kg intragastrisch in einem Volumen von 1 ml pro Ratte verabreicht wurden. Blutproben aus der Pfortader (250 µl) wurden zu bestimmten Zeiten in heparinisierten Röhrchen gesammelt; 10 µl Plasma wurde in Aquasol-Scintillationsflüssigkeit gelöst und in einem Scintillationszähler ausgezählt. Wie Tabelle 2 zu entnehmen ist, wurde die schlechteste Resorption mit tritiiertem Prostaglandin in Tris-Äthanol beobachtet, während tritiiertes Prostaglandin in Polyäthylenglykol 400 die beste Resorption zeigte.

Tabelle 1

| Inhaltsstoffe | Einheiten | Formulierung I | II |
|---|---|---|---|
| Tritiiertes Prostaglandin | mcg | 2,5 | 2,5 |
| Polyäthylenglykol 400 | mg | 400,0 | — |
| BHA | mg | 0,2 | 0,2 |
| Trimethylolaminomethan (Tris) | mg | — | 8 |
| Äthylalkohol q. s. | ml | — | 1 |
| Wasser q. s. | ml | 1 | — |

Tabelle 2

Bioverfügbarkeit von tritiiertem Prostaglandin in Ratten mit den Formulierungen I und II der Tabelle 1

| Formulierung | Fläche unter der Kurve ($10^4$ Zerfälle pro 10 µl Plasma | Plasma-Peak-Höhe $10^4$ Zerfälle pro 10 µl Plasma |
|---|---|---|
| I PEG 400 | 26,2 | 17 |
| II Tris-Äthanolgemisch | 12,8 | 4,3 |

Beispiel 8

Es wurden Dosierungsformulierungen A, B und C gemäß Tabelle 3 hergestellt. Die Formulierungen wurden an Hunde mit Heidenhain-Taschen verabreicht, die über Nacht gefastet hatten, wobei sie jedoch beliebig Zugang zu Wasser hatten. Zu Beginn wurden zwei Basisproben Magensaft in 15-Minuten-Abständen gesammelt, worauf mit einer Infusionsrate von 1 ml/Minute intravenös 20 µg/kg/Stunde Histaminhydrochlorid zur Erzeugung einer submaximalen Stimulation infundiert wurde. Die verschiedenen Formulierungen wurden 90 Minuten nach Beginn der Histaminhydrochloridinfusion oral verabreicht. Für die Dauer von 4 Stunden nach Verabreichung der Formulierungen wurde Magenaft in 15-Minuten-Intervallen gesammelt. Die Proben wurden auf pH, Volumen, gesamten Säuregehalt (meq/ml) und gesamte Säureausschüttung untersucht. Die Tabellen 4 und 5 zeigen die prozentuale Hemmung der Säureausschüttung (antisekretorischer Effekt) des Prostaglandins. Wie den Tabellen 4 und 5 zu entnehmen ist, ist die prozentuale Hemmung der Säureausschüttung überraschend hoch bei Formulierungen mit PEG und Ascorbylpalmitat im Vergleich zum Placebo (B) oder zur Formulierung C, die kein PEG enthält.

Tabelle 3

| | Formulierung A<br>Formulierungstyp Flüssig<br>mg/Kapsel | B<br>Placebo<br>mg/Kapsel | C<br>Fest<br>mg/Kapsel |
|---|---|---|---|
| Tritiiertes PGE+) | 0,25 | 0 | 0,25 |
| PEG 400 | 398,55 | 0 | 0 |
| Pluronic F-68*) | 0 | 200,0 | 200,0 |
| Lactose | 0 | 150,0 | 150,0 |
| Syloid 74**) | 0 | 25,0 | 25,0 |
| Primojel***) | 0 | 50,0 | 50,0 |
| Maisstärke | 0 | 20,0 | 20,0 |
| Talk | 0 | 20,0 | 20,0 |
| BHA | 0,2 | 0,2 | 0,2 |
| Ascorbylplamitat | 1,0 | 1,0 | 1,0 |
| | 400,0 | 466,20 | 466,45 |

*) Polyäthylen-Polyoxypropylen Copolymer
**) $SiO_2$
***) Modifizierte Stärke
+ 11R,16,16-Trimethyl-15R-hydroxy-9-oxoprosta-cis-5-trans-13-dien-1-säure

Tabelle 4

| Minuten nach Verabreichung | Formulierung A | B | C |
|---|---|---|---|
| | Formulierungstyp | | |
| | Flüssig | Placebo | Fest |
| | µg Prostaglandin pro Kapsel | | |
| | 250 | 0 | 250 |
| | Säureausschüttung (% Hemmung) | | |
| 15 | 53 | 0 | 0 |
| 30 | 62 | 0 | 0 |
| 45 | 94 | 0 | 0 |
| 60 | 67 | 4 | 0 |
| 75 | 95 | 7 | 1 |
| 90 | 98 | 0 | 10 |
| 105 | 89 | 0 | 28 |
| 120 | 81 | 2 | 0 |
| 135 | 65 | 6 | 15 |
| 150 | 70 | 12 | 0 |
| 165 | 69 | 1 | 0 |
| 180 | 61 | 18 | 0 |
| 195 | 48 | 21 | 0 |
| 210 | 47 | 4 | 0 |
| 225 | 39 | 0 | 15 |

Tabelle 5

| Minuten nach Verabreichung | Formulierung A<br>Formulierungstyp<br>Flüssig<br>µg Prostaglandin pro Kapsel<br>250<br>Säureausschüttung (% Hemmung) | B<br><br>Placebo<br><br>0 | C<br><br>Fest<br><br>250 |
|---|---|---|---|
| 15 | 10 | 0 | 0 |
| 30 | 61 | 1 | 0 |
| 45 | 91 | 1 | 0 |
| 60 | 83 | 0 | 0 |
| 75 | 81 | 1 | 1 |
| 90 | 77 | 1 | 0 |
| 105 | 80 | 0 | 17 |
| 120 | 72 | 9 | 0 |
| 135 | 58 | 4 | 12 |
| 150 | 49 | 6 | 0 |
| 165 | 40 | 0 | 0 |
| 180 | 44 | 0 | 0 |
| 195 | 11 | 0 | 0 |
| 210 | 14 | 0 | 0 |
| 225 | 59 | 0 | |

Beispiel 9

Tabelle 6 zeigt die überraschende Stabilität von 11R,16,16-Trimethyl-15R-oxoprosta-9-cis-5-trans-13-dien-1-säure (0,5 oder 0,25 mg/Kapsel) in PEG 400 in Gegenwart verschiedener Stabilisatoren. Die Stabilität wurde bei Raumtemperatur (RT) und erhöhten Temperaturen ermittelt und ist angegeben als Prozentsatz des abgebauten Prostaglandins, der dünnschichtchromatographisch ermittelt wurde.

Die Resultate zeigen, daß Ascorbylpalmitat die Prostaglandine in der PEG 400-Formulierung stabilisiert.

Tabelle 6

| Stabilisator | Alterung | Abbau |
|---|---|---|
| Kein Stabilisator | 3 Monate/RT | 2,0 |
| Kein Stabilisator | 3 Monate/RT | 50,0 |
| BHA (0,5%) | 3 Monate/RT<br>3 Monate/45° C | 2,0<br>50,0 |
| BHT (0,5%) | 3 Monate/RT<br>3 Monate/45° C | 3—4<br>50,0 |
| Ascorbylpalmitat (0,05%) | 3 Monate/RT<br>3 Monate/45° C | 0,1<br>2,0 |
| BHA + Ascorbylpalmitat je 0,05% | 3 Monate/RT<br>3 Monate/45° C | 0,2<br>3,0 |
| BHA + BHT je 0,05% | 5 Tage/RT<br>5 Tage/55° C | 0,2<br>1,5 |
| BHA + BHT je 0,25% | 5 Tage/RT | 0,3 |
| BHA + Ascorbylpalmitat je 0,05% | 5 Tage/RT<br>5 Tage/55° C | 0,1<br>0,2 |
| BHA + Ascorbylpalmitat je 0,25% | 5 Tage/RT | 0,2 |
| BHA + $\alpha$-Tocopherol 0,25 & 1% | 5 Tage/RT<br>5 Tage/55° C | 0,2<br>3,0 |
| BHA + Ascorbinsäure*) je 0,25% | 5 Tage/RT<br>5 Tage/55° C | 0,2<br>0,2 |
| BHA + BHA + $\alpha$-Tocopherol | 5 Tage/RT<br>5 Tage/55° C | 0,2<br>3,0 |
| BHA + $\alpha$-Tocopherol + Propylgallat | 5 Tage/RT<br>5 Tage/55° C | 0,2<br>3,4 |
| BHA + BHT + Ascorbylpalmitat | 5 Tage/RT<br>5 Tage/55° C | 0,2<br>0,2 |
| BHA + Ascorbylpalmitat + $\alpha$-Tocopherol | 5 Tage/RT<br>5 Tage/55° C | 0,2<br>0,2 |
| BHA + Ascorbylpalmitat | 6 Monate/RT | 0,1 |
| BHA + Ascorbylpalmitat | 12 Monate/RT | 0,1 |

*)in der FR-A-2 209 544 als Antioxidans für Prostaglandin erwähnt

**Patentansprüche für die Vertragsstaaten: BE, CH, DE, FR, GB, IT, LI, LU, NL, SE**

1. Weichgelatinekapsel zur oralen Verabreichung, enthaltend eine therapeutisch wirksame Menge eines Prostaglandins der Formel

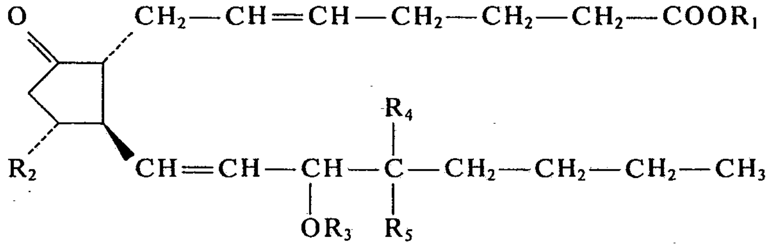

(I)

worin
$R_1$ Wasserstoff oder $C_{1-7}$-Alkyl;
$R_2$ Wasserstoff oder $C_{1-7}$-Alkyl;
$R_3$ Wasserstoff oder Acetyl;
$R_4$ Wasserstoff oder $C_{1-7}$-Alkyl; und
$R_5$ Wasserstoff, $C_{1-7}$-Alkyl oder Fluor darstellt,
als Wirkstoff; Polyoxyäthylensorbitanfettsäureester oder Polyäthylenglykole oder Gemische davon als Lösungsmittel; und eine wirksame Menge Ascorbylpalmitat als Stabilisator für das Prostaglandin.

2. Präparat gemäß Anspruch 1, dadurch gekennzeichnet, daß das Prostaglandin Nat-11R-methyl-16R-fluor-15R-hydroxy-9-oxoprosta-cis-5-trans-13-dien-1-säure. Nat-11R,16,16-trimethyl-15R-hydroxy-9-oxoprosta-cis-5-trans-13-dien-1-säure und dessen Methylester oder (8R,11R,12S,15R,5Z,13E)-15-(Acetoxy)-11,16,16-trimethyl-9-oxoprosta-cis-5-trans-13-dien-1-säure ist.

3. Präparat gemäß den Ansprüchen 1 oder 2, dadurch gekennzeichnet, daß das Lösungsmittel ein Polyäthylenglykol vom Molekulargewicht 200 bis 6000 ist.

4. Präparat gemäß Anspruch 3, dadurch gekennzeichnet, daß das Polyäthylenglykol ein Molekulargewicht von etwa 400 hat.

5. Präparat gemäß den Ansprüchen 1 oder 2, dadurch gekennzeichnet, daß das Lösungsmittel Polyoxyäthylen-sorbitan-stearat, -oleat oder -palmitat ist.

6. Präparat gemäß Anspruch 1, dadurch gekennzeichnet, daß das Lösungsmittel aus Polyäthylenglykol 400 und Polyäthylenglykol 4000 besteht.

**Patentansprüche für den Vertragsstaat: AT**

1. Verfahren zur Herstellung von Weichgelatinekapseln zur oralen Verabreichung, enthaltend eine therapeutisch wirksame Menge eines Prostaglandins der Formel

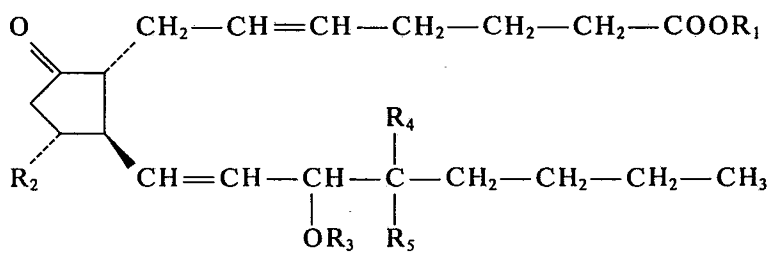

(I)

worin
$R_1$ Wasserstoff oder $C_{1-7}$-Alkyl;
$R_2$ Wasserstoff oder $C_{1-7}$-Alkyl;
$R_3$ Wasserstoff oder Acetyl;
$R_4$ Wasserstoff oder $C_{1-7}$-Alkyl; und
$R_5$ Wasserstoff, $C_{1-7}$-Alkyl oder Fluor darstellt,
als Wirkstoff; Polyoxyäthylensorbitanfettsäureester oder Polyäthylenglykole oder Gemische davon als Lösungsmittel; und eine wirksame Menge Ascorbylpalmitat als Stabilisator für das Prostaglandin, dadurch gekennzeichnet, daß man die Kapselinhaltstoffe mischt und die Mischung in Weichgelatinekapseln abfüllt.

2. Verfahren gemäß Anspruch 1, dadurch gekennzeichnet, daß das Prostaglandin Nat-11R-methyl-16R-fluor-15R-hydroxy-9-oxoprosta-cis-5-trans-13-dien-1-säure, Nat-11R,16,16-trimethyl-15R-hydroxy-9-oxoprosta-cis-5-trans-13-dien-1-säure und dessen Methylester oder (8R,11R,12S,15R,5Z,13E)-15-(Acetoxy)-11,16,16-trimethyl-9-oxoprosta-cis-5-trans-13-dien-1-säure ist.

3. Verfahren gemäß den Ansprüchen 1 oder 2, dadurch gekennzeichnet, daß das Lösungsmittel ein Polyäthylenglykol vom Molekulargewicht 200 bis 6000 ist.

4. Verfahren gemäß Anspruch 3, dadurch gekennzeichnet, daß das Polyäthylenglykol ein Molekulargewicht von etwa 400 hat.

5. Verfahren gemäß den Ansprüchen 1 oder 2, dadurch gekennzeichnet, daß das Lösungsmittel Polyoxyäthylen-sorbitan-stearat, -oleat oder -palmitat ist.

6. Verfahren gemäß Anspruch 1, dadurch gekennzeichnet, daß das Lösungsmittel aus Polyäthylenglykol 400 und Polyäthylenglykol 4000 besteht.

**Claims for the Contracting States: BE, CH, DE, FR, GB, IT, LI, LU, NL, SE**

1. A soft gelatin capsule for oral administration containing a therapeutically effective amount of a prostaglandin of the formula

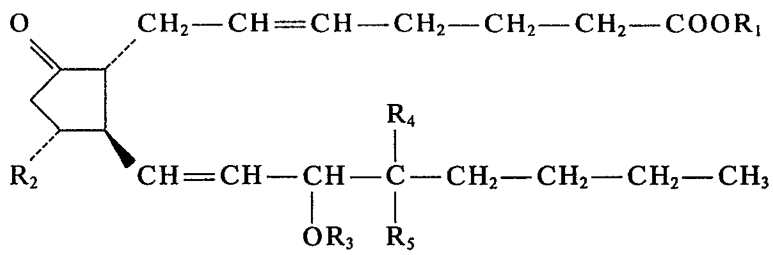

(I)

wherein

$R_1$ represents hydrogen or $C_{1-7}$-alkyl;
$R_2$ represents hydrogen or $C_{1-7}$-alkyl;
$R_3$ represents hydrogen or acetyl;
$R_4$ represents hydrogen or $C_{1-7}$-alkyl; and
$R_5$ represents hydrogen, $C_{1-7}$-alkyl or fluoro,

as the active substance; polyoxyethylene sorbitan fatty acid esters or polyethylene glycols or mixture thereof as the solvent; and an effective amount of ascorbyl palmitate as a stabilizer for the prostaglandin.

2. A preparation in accordance with claim 1, characterized in that the prostaglandin is nat-11R-methyl-16R-fluoro-15R-hydroxy-9-oxoprosta-cis-5-trans-13-dien-1-oic acid, nat-11R,16,16-trimethyl-15R-hydroxy-9-oxoprosta-cis-5-trans-13-dien-1-oic acid and its methyl ester or (8R,11R,12S,15R,5Z,13E)-15-(acetoxy)-11,16,16-trimethyl-9-oxoprosta-cis-5-trans-13-dien-1-oic acid.

3. A preparation in accordance with claims 1 or 2, characterized in that the solvent is a polyethylene glycol of molecular weight 200 to 6000.

4. A preparation in accordance with claim 3, characterized in that the polyethylene glycol has a molecular weight of about 400.

5. A preparation in accordance with claims 1 or 2, characterized in that the solvent is polyoxyethylene sorbitan stearate, polyoxyethylene sorbitan oleate or polyoxyethylene sorbitan palmitate.

6. A preparation in accordance with claim 1, characterized in that the solvent consists of polyethylene glycol 400 and polyethylene glycol 4000.

**Patent Claims for the Contracting State: AT**

1. A process for the manufacture of soft gelatin capsules for oral administration containing a therapeutically effective amount of a prostaglandin of the formula

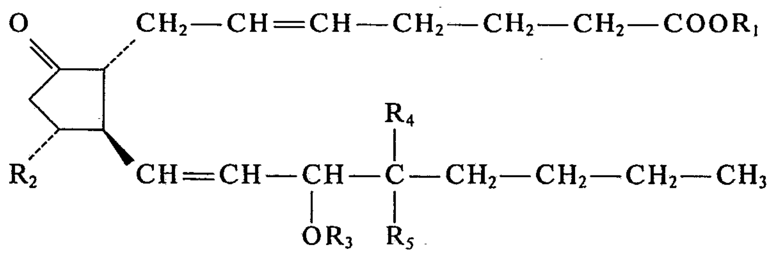

(I)

wherein
$R_1$ represents hydrogen or $C_{1-7}$-alkyl;
$R_2$ represents hydrogen or $C_{1-7}$-alkyl;
$R_3$ represents hydrogen or acetyl;
$R_4$ represents hydrogen or $C_{1-7}$-alkyl; and
$R_5$ represents hydrogen, $C_{1-7}$-alkyl or fluoro,
as the active substance; polyoxyethylene sorbitan fatty acid esters or polyethylene glycols or mixture thereof as the solvent; and an effective amount of ascorbyl palmitate as a stabilizer for the prostaglandin, characterized by mixing the capsule ingredients and filling the mixture into soft gelatin capsules.

2. A process in accordance with claim 1, characterized in that the prostaglandin is nat-11R-methyl-16R-fluoro-15R-hydroxy-9-oxoprosta-cis-5-trans-13-dien-1-oic acid, nat-11R,16,16-trimethyl-15R-hydroxy-9-oxoprosta-cis-5-trans-13-dien-1-oic acid and its methyl ester or (8R,11R,12S,15R,5Z,13E)-15-(acetoxy)-11,16,16-trimethyl-9-oxoprosta-cis-5-trans-13-dien-1-oic acid.

3. A process in accordance with claims 1 or 2, characterized in that the solvent is a polyethylene glycol of molecular weight 200 to 6000.

4. A process in accordance with claim 3, characterized in that the polyethylene glycol has a molecular weight of about 400.

5. A process in accordance with claims 1 or 2, characterized in that the solvent is polyoxyethylene sorbitan stearate, polyoxyethylene sorbitan oleate or polyoxyethylene sorbitan palmitate.

6. A process in accordance with claim 1, characterized in that the solvent consists of polyethylene glycol 400 and polyethylene glycol 4000.

**Revendications pour l'Etat contractant: AT**

1. Procédé de préparation de capsules de gélatine molle pour l'administration orale, contenant une quantité thérapeutique efficace d'une prostaglandine de formule:

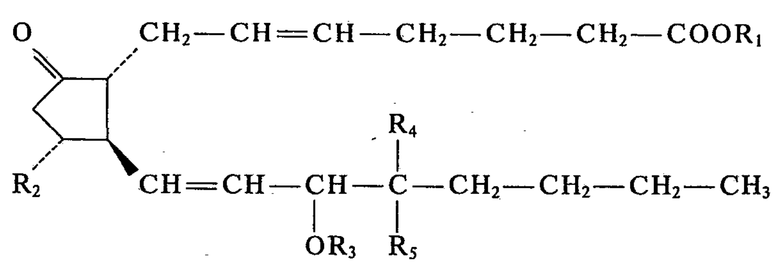

(I)

dans laquelle
$R_1$ représente l'hydrogène ou un groupe alkyle en $C_1-C_7$;
$R_2$ représente l'hydrogène ou un groupe alkyle en $C_1-C_7$;
$R_3$ représente l'hydrogène ou un groupe acétyle;
$R_4$ représente l'hydrogène ou un groupe alkyle en $C_1-C_7$ et
$R_5$ représente l'hydrogène ou un groupe alkyle en $C_1-C_7$ ou le fluor
en tant que substance active, des esters d'acides gras et de sorbitanne polyoxyéthylène ou des polyéthylène-glycols ou des mélanges de ces substances en tant que solvant; et une quantité efficace de palmitate d'ascorbyle en tant que stabilisant de la prostaglandine, caractérisé en ce que l'on mélange les constituants de la capsule et on introduit le mélange dans des capsules de gélatine molle.

2. Procédé selon la revendication 1, caractérisé en ce que la prostaglandine est l'acide Nat-11R-méthyl-16R-fluoro-15R-hydroxy-9-oxoprosta-cis-5-trans-13-diène-1-oïque, l'acide Nat-11R,16,16-trimé-

thyl-15R-hydroxy-9-oxoprosta-cis-5-trans-13-diène-1-oïque et son ester méthylique ou l'acide (8R,11R,12S,15R,5Z,13E)-15-(acétoxy)-11,16,16-triméthyl-9-oxoprosta-cis-5-trans-13-diène-1-oïque.

3. Procédé selon les revendications 1 ou 2, caractérisé en ce que le solvant est un polyéthylène-glycol de poids moléculaire 200 à 6000.

4. Procédé selon la revendication 3, caractérisé en ce que le polyéthylène-glycol présente un poids moléculaire d'environ 400.

5. Procédé selon les revendications 1 ou 2, caractérisé en ce que le solvant consiste en stéarate, oléate ou palmitate de sorbitanne polyoxyéthyléné.

6. Procédé selon la revendication 1, caractérisé en ce que le solvant consiste en polyéthylène-glycol 400 et polyéthylène-glycol 4000.

**Revendications pour les Etats contractants: BE, CH, DE, FR, GB, IT, LI, LU, NL, SE**

1. Capsule de gélatine molle pour l'administration orale, contenant une quantité thérapeutique efficace d'une prostaglandine de formule

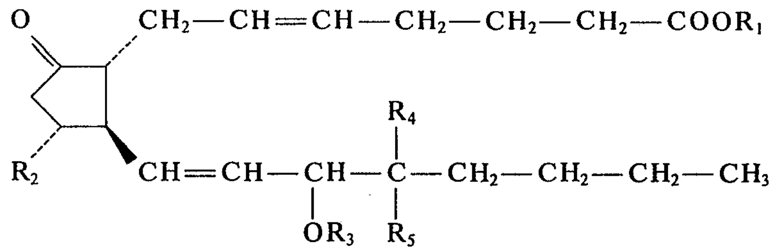

(I)

dans laquelle

$R_1$ représente l'hydrogène ou un groupe alkyle en $C_1-C_7$;
$R_2$ représente l'hydrogène ou un groupe alkyle en $C_1-C_7$;
$R_3$ représente l'hydrogène ou un groupe acétyle;
$R_4$ représente l'hydrogène ou un groupe alkyle en $C_1-C_7$ et
$R_5$ représente l'hydrogène ou un groupe alkyle en $C_1-C_7$ ou le fluor

en tant que substance active; des esters d'acides gras et de sorbitanne polyoxyéthylène ou des polyéthylène-glycols ou des mélanges de ces substances en tant que solvant; et une quantité efficace de palmitate d'ascorbyle en tant que stabilisant de la prostaglandine.

2. Préparation selon la revendication 1, caractérisée en ce que la prostaglandine est l'acide Nat-11R-méthyl-16R-fluoro-15R-hydroxy-9-oxoprosta-cis-5-trans-13-diène-1-oïque, l'acide Nat-11R,16,16-triméthyl-15R-hydroxy-9-oxoprosta-cis-5-trans-13-diène-1-oïque et son ester méthylique ou l'acide (8R,11R,12S,15R,5Z,13E)-15-(acétoxy)-11,16,16-triméthyl-9-oxoprosta-cis-5-trans-13-diène-1-oïque.

3. Préparation selon les revendications 1 ou 2, caractérisée en ce que le solvant est un polyéthylène-glycol de poids moléculaire 200 à 6000.

4. Préparation selon la revendication 3, caractérisée en ce que le polyéthylène-glycol présente un poids moléculaire d'environ 400.

5. Préparation selon les revendications 1 ou 2, caractérisée en ce que le solvant est du stéarate, de l'oléate ou du palmitate de sorbitanne polyoxyéthyléné.

6. Préparation selon la revendication 1, caractérisée en ce que le solvant consiste en polyéthylène-glycol 400 et polyéthylène-glycol 4000.